# EUROPEAN PATENT APPLICATION

(11) **EP 3 998 250 A2**
(43) Date of publication of application: **18.05.2022**
(21) Application number: 21460046.2
(22) Date of filing: 21.12.2021
(51) Int. Cl.: C07C 211/63, B01J 31/02

(54) **A METHOD FOR PREPARING MONO, DI- TRI-, TETRA-, PENTA- OR HEXACATIONIC PROTIC IONIC LIQUIDS**

(30) Priority: 22.12.2020 PL 43644320
(71) Applicant: Politechnika Slaska, 44-100 Gliwice (PL)
(72) Inventor: Brzeczek-Szafran, Alina, 40-018 Katowice (PL); Chrobok, Anna, 42-674 Zbroslawice (PL); Szelwicka, Anna, 41-800 Zabrze (PL); Barteczko, Natalia, 44-292 Rybnik (PL); Wleclawik, Justyna, 42-260 Rudnik (PL)

(57) **Abstract**

For preparing mono, di- tri-, tetra-, penta- or hexacationic protic ionic liquids, wherein the sulfuric acid concentration is 30 - 98%, preferably 95 - 98%, is reacted with aliphatic diamine, triamine, tetraamine, pentaamine or hexaamine, with the mole ratio of 1 amine group in amine molecule to sulfuric acid ranging from 1:1 to 1:5, the resultant mixture is stirred in the temperature of 50 - 150°C for 1 - 2 hours, dried under reduced pressure of at least 50 mbar at 20 - 150°C for at least 1 hour, preferably 8 hours.

## Description

This invention describes a method for the preparation of protic di- tri-, tetra-, penta-, or hexacationic ionic liquids, as acidic catalysts, dedicated for the purpose of organic transformations (especially esterification), Beckmann rearrangement or alkylation.

The use of conventional acids (H₂SO₄, HF or AlCl₃) is often associated with problems such as corrosiveness, toxicity or the generation of large amounts of waste. Waste generation is the result of the difficulty of separating the catalyst from the reaction mixture after the process, so that the catalyst can be recycled rather than wasted. An additional limitation may be the insufficient solubility of substrates in liquid acids.

Protic ionic liquids obtained by proton transfer from Brønsted acid to Brønsted base are an attractive group of acid catalysts and solvents due to their simplicity in synthesis, low cost and the possibility of modifying the system's acidic properties by convenient modification of molar ratios of the reagents. In many reaction systems, protic ionic liquids constitute a separate phase, which allows them to be easily separated from the reaction mixture and reused in the next reaction cycle, thus reducing the amount of waste generated in the process (Vafaeezadeh, Alinezhad J. Mol. Liq. 2016, 218, 95-105). According to the estimates from 2014, the production cost of imidazolium hydrogen sulfate is within the range of 2.96-5.88 USD/kg, while triethylammonium hydrogen sulfate is 1.24 USD/kg (Chen et.al. Green Chem. 2014, 16, 3098-3106). These prices are comparable to production cost of commonly used organic solvents such as acetone and ethyl acetate, which varies between 1.30 and 1.40 USD/kg.

According to the latest estimates, with consideration to the production costs of protic ionic liquids and the costs related to their environmental impact (production, use and disposal), the gross cost of producing triethylammonium hydrogen sulfate and imidazolium hydrogen sulfate is equal to $ 0.78/kg and $ 1.46/kg respectively (Baaqel et al. Green Chem. 2020, 22, 3132-3140), making them suitable alternatives to organic solvents.

Numerous patents have been developed for the preparation of protic ionic liquids.

The patent description in document WO0016902A1 describes a method of obtaining protic ionic liquids by neutralizing aliphatic and aromatic monoamines with organic and inorganic acids. The invention relates to the use of the protic ionic liquids as acidic solvents and catalysts for use in processes that use acid catalysis.

An Australian patent AU2011202949A1 discloses a method of obtaining protic ionic liquids using primary, secondary, and tertiary monoamines. In addition, the patent literature mentions the use of protic ionic liquids in processes of industrial importance, e.g., alkylation (US2010331599A1), esterification (CN106939014A) and biomass dissolution (CN105316375A).

In the wider scientific literature (Matuszek et al. Green Chem., 2014, 16, 3463-3471) methods of increasing the acidity of protic ionic liquids with a hydrogen sulfate anion are described, involving the use of sulfuric acid in excess by two and three-folds relative to the amine.

A Polish patent (PL231053) presents the synthesis of protic ionic liquids based on oligomeric anions of the general formula 2 [X]⁺[(*HSO*₄)(*H*₂*SO*₄)*ₓ*]⁻ wherein subscript *x* = 0, 1, 2, 3, and X is the cation. Cations mentioned were: N,N,N-trialkylammonium cation [NR₁R₂R₃]⁺, wherein R₁, R₂, R₃ = C₁-C₂₀; N-alkylpyrolidinium cation [RPyr]⁺, wherein R = C₁-C₂₀; N-alkylimidazolium cation [HRIm]⁺, wherein R = C₁- C₂₀; O-alkylpyridinium cation [Ro-Py]⁺; m-alkylpyridinium cation [Rm-Py]⁺; p-alkylpyridinium cation [Rp-Py]⁺; wherein R = C₁-C₂₀. These acidic protic ionic liquids can be used in the preparation of amides by following a Beckmann rearrangement, and in the preparation of esters of succinic acid (protocol described in the Polish patent PL234515), esters of phthalic acid and terephthalic acid (protocol described in the Polish patent PL234516) or levulinic acid (protocol described in the Polish patent PL232790).

In addition, the patent literature describes methods of obtaining protic ionic liquids used for fuel desulfurization, where an excess of organic or inorganic acid relative to the amine is used, with ratios ranging from 1: 1 to 9: 1 (CN105694945A). Mixtures of sulfuric acid with an ionic liquid that has Brønsted acidity are also described in a US patent (US2010331599A1), where sulfuric acid constitutes at least 50% by weight of the composition of the ionic liquid, with the cation being one of the following: 1-butyl-3-methylimidazolium; 1-methyl-3-(butyl-4-sulfonate)imidazolium; 1-butyl-3-(butyl-3-sulfonyl)imidazolium; or 1-octyl-3-methylimidazolium.

During research on the development of protic acid catalysts that use an excess of sulfuric acid relative to the amine, it was found that similar catalytic and physical properties were obtained, compared to those observed for liquids based on the following cations: trialkylammonium monocation [NR₁R₂R₃]⁺, wherein R₁, R₂, R₃ = C₁-C₂₀; N-alkylpyrolidinium [RPyr]⁺, wherein R = C₁-C₂₀; N-alkylimidazolium [HRIm]⁺, wherein R = C₁-C₂₀; O-alkylpyridinium [Ro-Py]⁺; m-alkylpyridinium [Rm-Py]⁺; *p*-alkylpyridinium [Rp-Py]⁺, wherein R = C₁- C₂₀; and the hydrogen sulfate anion, with a general formula of [(HSO₄)(H₂SO₄)ₓ], wherein subscript x = 1, 2, 3, and the cation of the protic ionic liquid is an amine, containing at least two amine functional groups in the structure.

The use of aliphatic or aromatic diamines, triamines, tetraamines, pentaamines or hexaamines (with an excess of sulfuric acid with a ratio ranging from 1: 1 to 5: 1 relative to 1 amine group in the amine molecule) allows an increase to the content of sulfuric acid in the protic ionic liquid composition. By comparison with the ionic liquid synthesized using monoamine, the polyamines are more cost effective.

The method for preparing mono, di- tri-, tetra-, penta- or hexacationic protic ionic liquids, involving using a concentration of sulfuric acid ranging from 30 - 98%. It is preferable to use 95 - 98% to react with aliphatic diamine, triamine, tetramine, pentamine or hexaamine. The reaction uses a molar ratio of 1 amine group in the amine molecule to sulfuric acid, ranging from 1:1 to 1:5. The resultant mixture is stirred at a temperature of 50 - 150°C for 1 - 2 hours, then dried under reduced pressure at 50 mbar at 20°C - 150°C for at least 1 hour, preferably 8 hours.

Preferably in the invention a diamine of a general formula NR₁R₂R₃NR₄R₅ is used, where R₁₋₅ = H, C₁-C₂₀, wherein the molar ratio of diamine to sulfuric acid ranges from 1:2 to 1:10. Preferably in the invention a triamine of a general formula NR₁R₂R₃NR₄R₅NR₆R₇ is used, where R₁₋₇ = H, C₁-C₂₀, and wherein the molar ratio of triamine to sulfuric acid ranges from 1:3 to 1:15.

Preferably in the invention a tetraamine of a general formula NR₁R₂R₃NR₄R₅NR₆R₇NR₈R₉ is used, where R₁₋₉ = H, C₁- C₂₀, and wherein the molar ratio of tetra-amine to sulfuric acid ranges from 1:4 to 1:20.

Preferably in the invention a pentaamine of a general formula NR₁R₂R₃NR₄R₅NR₆R₇NR₈R₉NR₁₀R₁₁ is used, where R₁₋₁₁ = H, C₁-C₂₀, and wherein the molar ratio of penta-amine to sulfuric acid ranges from 1:5 to 1:25.

Preferably in the invention a hexaamine of a general formula NR₁R₂R₃NR₄R₅NR₆R₇NR₈R₉NR₁₀R₁₁NR₁₂R₁₃ is used, where R₁₋₁₃ = H, C₁-C₂₀, and wherein the molar ratio of hexamine to sulfuric acid ranges from 1:6 to 1:30.

Preferably a diamine is selected from N,N-dimethylaminopropylamine, hexamethylenediamine.

Preferably a triamine is selected from diethylenetriamine, bis(hexamethylene)triamine. Preferably a tetraamine is triethylenetetramine.

Preferably a pentaamine is tetraethylenepentamine.

Preferably a hexaamine is pentaethylenehexamine.

The advantage of the solution according to the invention is that it is a low cost and simple procedure for the preparation of acidic catalysts based on sulfuric acid and di-, tri-, tetra-, penta- or hexaamines, which can be used as a separate reaction phase in selected reaction systems. The protic ionic liquids for their production in which sulfuric acid is used in various stoichiometric amounts, constitutes an attractive alternative to the traditionally used Brønsted acids, which most often dissolve in the reaction mixture and prevents their easy separation and re-use. Acidity, viscosity, and density of the ionic liquids described in the invention can be modified by the amount of sulfuric acid used to prepare the ionic liquid.

The present invention is further illustrated with reference to the following Examples:

### Example 1

Sulfuric acid (98%, 167.72 g) is introduced into a 500 ml round bottom flask. Then, while stirring the acid in an ice bath, 85.62 g of *N*,*N*-dimethylaminopropylamine is slowly added dropwise, keeping the molar ratio of an amine to an acid at 1: 2. The reaction mixture is stirred for 1 hour at 60°C until a homogeneous light brown mixture is obtained. The ionic liquid is then dried under reduced pressure of 100 Pa at 50°C for 8 hours. The yielded ionic liquid has a kinematic viscosity of 11040 mm²/s and density of 1.50 kg/m³ at 25°C.

### Example 2

Sulfuric acid (98%, 186.74 g) is introduced into a 500 ml round bottom flask. Then, while stirring the acid in an ice bath, 66.98 g of bis(hexamethylene)triamine is slowly added dropwise, keeping the molar ratio of an amine to an acid of 1: 6. The reaction mixture is stirred for 1 h at 80°C until a homogeneous dark brown mixture was obtained. The ionic liquid is then dried under reduced pressure of 100 Pa at 50°C for 8 hours. The yielded ionic liquid has a kinematic viscosity of 688.48 mm²/s and density of 1.55 kg/m³ at 25°C.

### Example 3

Sulfuric acid (98%, 228.32 g) is introduced into a 500 ml round bottom flask. Then, while stirring the acid in an ice bath, 26.16 g of diethylenetriamine is slowly added dropwise, keeping the molar ratio of an amine to an acid of 1: 9. The reaction mixture is stirred for 1 hour at 80°C until a homogeneous colorless mixture is obtained. The ionic liquid is then dried under reduced pressure of 100 Pa at 50°C for 8 hours. The yielded ionic liquid has a kinematic viscosity of 112.17 mm²/s and density of 1.84 kg/m³ at 25°C.

## Claims

1. For preparing mono, di- tri-, tetra-, penta- or hexacationic protic ionic liquids, wherein the sulfuric acid concentration is 30 - 98%, preferably 95 - 98%, is reacted with aliphatic diamine, triamine, tetraamine, pentaamine or hexaamine, with the mole ratio of 1 amine group in amine molecule to sulfuric acid ranging from 1:1 to 1:5, the resultant mixture is stirred in the temperature of 50 - 150°C for 1 - 2 hours, dried under reduced pressure of at least 50 mbar at 20 - 150°C for at least 1 hour, preferably 8 hours.

2. A diamine as in claim 1 is of a general formula NR₁R₂R₃NR₄R₅, where R₁₋₅ = H, C₁-C₂₀, and wherein the molar ratio of diamine to sulfuric acid ranges from 1:2 - 1:10.

3. A triamine as in claim 1 is of a general formula NR₁R₂R₃NR₄R₅NR₆R₇, where R₁₋₇ = H, C₁-C₂₀, and wherein the molar ratio of triamine to sulfuric acid ranges from 1:3 - 1:15.

4. A tetraamine as in claim 1 is of a general formula NR₁R₂R₃NR₄R₅NR₆R₇NR₈R₉, where R₁₋₉ = H, C₁- C₂₀, and wherein the molar ratio of tetraamine to sulfuric acid ranges from 1:4 to 1:20.

5. A pentaamine as in claim 1 is of the general formula NR₁R₂R₃NR₄R₅NR₆R₇NR₈R₉NR₁₀R₁₁, where R₁₋₁₁ = H, C₁- C₂₀, and wherein the molar ratio of pentaamine to sulfuric acid ranges from 1:5 - 1:25.

6. A hexaamine as in claim 1 is of a general formula NR₁R₂R₃NR₄R₅NR₆R₇NR₈R₉NR₁₀R₁₁NR₁₂R₁₃, where R₁₋₁₃ = H, C₁-C₂₀, and wherein the molar ratio of hexaamine to sulfuric acid ranges from 1:6 - 1:30.

7. A diamine as claimed in claim 1 is N,N-dimethylaminopropylamine, hexamethylenediamine.

8. A triamine as claimed in claim 1 is diethylenetriamine, bis(hexamethylene)triamine.

9. A tetraamine as claimed in claim 1 is triethylenetetramine.

10. A pentaamine as claimed in claim 1 is tetraethylenepentamine.

11. A hexaamine as claimed in claim 1 is pentaethylenehexamine.
